# EUROPÄISCHE PATENTSCHRIFT

(11) **EP 1 071 481 B1**
(45) Veröffentlichungstag und Bekanntmachung des Hinweises auf die Patenterteilung: **02.10.2002**
(21) Anmeldenummer: 99920690.7
(22) Anmeldetag: 16.04.1999
(51) Int. Cl.: A61L 24/00, A61P 19/00

(54) **ZUBEREITUNG ZUR KOLLAGENNEUBILDUNG**
PREPARATION FOR REGENERATING COLLAGEN
PREPARATION DE REGENERATION DE COLLAGENE

(30) Priorität: 16.04.1998 DE 19816934; 29.05.1998 US 87342 P; 21.10.1998 DE 19848597
(43) Veröffentlichungstag der Anmeldung: 31.01.2001
(73) Patentinhaber: Dietz, Georg, Prof. Dr., D-81925 München (DE)
(72) Erfinder: Dietz, Georg, Prof. Dr., D-81925 München (DE)
(74) Vertreter: Henkel, Feiler, Hänzel
(86) Internationale Anmeldenummer: EP9902582
(87) Internationale Veröffentlichungsnummer: WO99053969

(56) Entgegenhaltungen:
- EP-A- 0 464 545
- EP-A- 0 908 169
- WO-A-93/24154
- WO-A-97/42269
- DE-C- 4 240 713
- PATENT ABSTRACTS OF JAPAN vol. 012, no. 258 (C-513), 20. Juli 1988 (1988-07-20) & JP 63 044506 A (SHOWA YAKUHIN KAKO KK), 25. Februar 1988 (1988-02-25)

## Beschreibung

Die Erfindung betrifft eine Zubereitung aus einem fetten Öl vegetarischen oder animalischen Ursprungs, Calciumhydroxid, einem zwei- oder mehrwertigen Alkohol sowie gegebenenfalls pharmazeutisch verträglichen Hilfsstoffen, die Herstellung eines derartigen Gemischs, sowie die Verwendung eines derartigen Gemischs bei der Herstellung eines Medikaments zur Förderung der Kollagenneubildung in vivo.

Der Knochen besteht zu etwa 60 % aus mineralischer Substanz (Hydroxylapatit, Calciumphosphat) und etwa 40 % aus organischem Material, vor allem Kollagen. Der Knochenstoffwechsel wird hauptsächlich durch das Zusammenspiel von Knochen aufbauenden Zellen (Osteoblasten) und Knochen abbauenden Zellen (Osteoklasten und Osteozyten), deren Aktivitäten im gesunden Knochen in einem ausgewogenen Verhältnis stehen, bestimmt.

Die Knochenbildung läßt sich in zwei Hauptphasen gliedern, (a) die Synthese von organischem Gewebe (Kollagensynthese) und (b) die daran anschließende und durch sogenannte Matrixvesikel vermittelte Einlagerung von mineralischer Substanz in die vorgegebene organische Matrix.

Das Bindegewebsprotein Kollagen macht den größten Anteil der organischen Substanz des Knochens aus. Das Protein besteht aus drei helikal gewundenen Polypeptidketten, deren Aminosäurezusammensetzung variieren kann, was zu einer Vielfalt einzelner Kollagentypen führt. Allen Kollagentypen gemeinsam ist eine außerordentlich hohe mechanische Festigkeit der Kollagenfaser. Diese Festigkeit beruht auf einer Vielzahl von intra- und intermolekularen Bindungen der Kollagenfasern, welche auf diese Weise das dichte Kollagenfasernetzwerk des Bindegewebes bilden. Knochengewebe wird - wie bereits erwähnt - durch Einlagerung von mineralischen Substanzen (Hydroxylapatit und Calciumphosphat) in dieses Netzwerk gebildet. Jedem Knochenaufbau infolge von Wachstums- oder Regenerationsprozessen geht eine Kollagenbiosynthese voraus.

Bislang überläßt man bei Knochentraumen beliebiger Genese den Knochenneubildungsprozeß sich selbst, unterstützt ihn allenfalls mit Antibiotika und Corticoiden, um einer den Heilungsprozeß störenden eventuellen Infektionsgefahr vorzubeugen.

Es sind auch mehrere Faktoren beschrieben worden, welche die Knochenbildung und -regeneration beeinflussen können. Hauptsächlich handelt es sich hierbei um physikalische Faktoren (mechanische und elektrische Kräfte), Hormone (z.B. Parathormon, Calcitonin, Insulin, Glucocorticoide, 1,25,OH,D3) und eine nicht genau umrissene Gruppe von Wachstumsfaktoren mit Proceincharakter (Osteochinin, Osteonektin, "insulinartige Wachstumsfaktoren") - vgl. S. Wallach, L.V. Avioli, J.H. Carstens jun. "Factors in Bone Formation", Calcified Tissue International 45: 4-6 (1989)). Der Einfluß der Wasserstoffionen-Konzentration (pH-Wert) auf die Stoffwechselprozesse bei der Knochenregeneration wurde bislang noch nicht ausreichend untersucht.

Dietz beschreibt in der DE-A-42 40 713 die Verwendung eines Gemisches aus Calciumhydroxid und Oleum pedum tauri bei der Kollagenneubildung im Zuge von Knochentraumen. Diese Zubereitung aus Calciumhydroxid und Oleum pedum tauri krankt jedoch daran, daß seine Haltbarkeit infolge Saponifizierung stark eingeschränkt ist. Hierdurch kann es zu einer Beeinträchtigung der Wirkung des Gemisches kommen.

Der Erfindung lag folglich die Aufgabe zugrunde, ein verbessertes Gemisch mit langer Haltbarkeit zur gezielten externen Beeinflussung des Knochenneubildungs- oder regenerationsprozesses durch Stimulierung oder Einleitung der Kollagenneubildung anzugeben.

Es hat sich nun überraschenderweise gezeigt, daß durch Verwendung einer Zubereitung aus Calciumhydroxid, einem zwei- oder mehrwertigen Alkohol und einem fetten Öl vegetarischen oder animalischen Ursprungs sowie gegebenenfalls pharmazeutisch verträglichen Hilfsstoffen die Haltbarkeit der Zubereitung deutlich verbessert werden kann und dadurch bei Verwendung dieser Zubereitung in bzw. bei Knochentraumen eine in ihrem Umfang bessere Kollagenneubildung in vivo erfolgt.

Gegenstand der vorliegenden Erfindung sind somit Zubereitungen, die Calciumhydroxid, einen zwei- oder mehrwertigen Alkohol und ein fettes Öl vegetarischen oder animalischen Ursprungs sowie gegebenenfalls pharmazeutisch verträgliche Hilfsstoffe enthalten.

Gegenstand der vorliegenden Erfindung ist ferner ein Verfahren zur Herstellung einer derartigen Zubereitung durch Einmischen des Calciumhydroxids und des zwei- oder mehrwertigen Alkohols sowie gegebenenfalls von pharmazeutisch verträglichen Hilfsstoffen in ein fettes Öl vegetarischen oder animalischen Ursprungs.

Gegenstand der vorliegenden Erfindung ist ferner die Verwendung einer derartigen Zubereitung bei der Herstellung eines Medikaments zur Förderung der Kollagenneubildung in vivo.

Bariumsulfathaltige Gemische aus Calciumhydroxid und Oleum pedum tauri wurden in der Zahnmedizin als Wurzelfüllpaste verwendet (DE-PS 29 32 738). Gemische aus Carboxylatzement, Calciumhydroxid und Oleum pedum tauri wurden ebenfalls in der Zahnmedizin bereits als temporäre Befestigungsmittel für provisorische Zahnstumpfabdeckungen verwendet (DE-PS 34 13 864). Aufgabe des Calciumhydroxids in ersterem Fall ist es, das saure Milieu in den Wurzelkanälen ins Alkalische umzustimmen mit der Folge der Beseitigung von Entzündungen und allmählicher Bildung einer Hartgewebsbarriere. In letzerem Falle wird die Pulpitisprophylaktische Wirkung von Calciumhydroxid ausgenutzt. Das Oleum pedum tauri dient in beiden Fällen als Anteigmittel, um einerseits eine einfache und vollständige Füllung der Wurzelkanäle mit dem eigentlichen Wirkstoff Calciumhydroxid (und dem Kontrastmittel Bariumsulfat) zu gewährleisten und andererseits die Aushärtung des temporären Befestigungsmittels für provisorische Zahnstumpfabdeckungen soweit zu verlangsamen, daß das Calciumhydroxid noch durch die feinen Dentinkanälchen zur Pulpa vordringen und dort seine Wirkung entfalten kann. In beiden Literaturstellen findet sich nicht der geringste Hinweis darauf, daß die erfindungsgemäße Mischung eine massive Kollagenneubildung als Voraussetzung für eine Knochenregeneration zu induzieren vermag.

Der erfindungsgemäß neuer und im folgenden verwendete Ausdruck "Zubereitung" bezeichnet eine pharmazeutische Zubereitung (im folgenden manchmal auch als Gemisch bezeichnet), die mindestens die oben genannten Bestandteile enthält. Sie eignet sich insbesondere zur Verabreichung an Menschen oder Tiere zur Forschung der Kollagenneubildung als Voraussetzung einer Knochenneubildung bzw Knochenregeneration.

Im folgenden werden die Bestandteile des erfindungsgemäßen Gemischs detaillierter beschrieben:
Bei den verwendbaren fetten Ölen vegetarischen Ursprungs kann es sich um einen oder mehrere Bestandteile der folgenden vegetarischen Öle handeln:
   Soja-, Sonnenblumen-, Rüb-, Baumwollsaat-, Lein-, Rizinus-, Palm-, Palmkern-, Kokos- und Olivenöle.
   Bevorzugt handelt es sich bei den verwendbaren fetten vegetarischen Ölen um fette vegetarische Öle mit hoher Stabilität bei Erhitzung, wie Soja-, Sonnenblumen- und Olivenöle, insbesondere um Olivenöl.
   Bei den verwendbaren fetten tierischen Ölen kann es sich um einen oder mehrere Bestandteile der folgenden animalischen Öle handeln:
      Fisch-, Klauenöle und Talge.

Bevorzugt handelt es sich bei den verwendbaren animalischen Ölen um Klauenöle, insbesondere um Oleum pedum tauri.

Bei den verwendbaren zwei- oder mehrwertigen Alkoholen kann es sich um zweiwertige Alkohole, wie Ethylenglykol, Propylenglykol, Butylenglykol, Pentylenglykol, Hexylenglykol sowie Polyethylenglykole, wie Diethylenglykol, Triethylenglykol, Polypropylenglykole, wie Dipropylenglykol, dreiwertige Alkohole wie Glycerin, vierwertige Alkohole wie Threit, Erythrit, fünfwertige Alkohole, wie Arabit, Adonit, Xylit, sechswertige Alkohole, wie Sorbit, Mannit, Dulcit, oder höherwertige Alkohole handeln.

Bevorzugt verwendet als zwei- oder mehrwertiger Alkohol werden zwei- und dreiwertige Alkohole, wie Ethylenglykol, Propylenglykol, Butylenglykol, Pentylenglykol, Hexylenglykol sowie Polyethylenglykole, wie Diethylenglykol, Triethylenglykol, Polypropylenglykole, wie Dipropylenglykol, sowie dreiwertige Alkohole wie Glycerin, insbesondere Glycerin.

Ohne an eine Theorie gebunden werden zu wollen, gehen wir davon aus, daß der zwei- oder mehrwertige Alkohol eine Saponifizierung des vegetarischen oder animalischen fetten Öls verhindert. Dadurch kann das Gemisch länger in einer knetbaren oder cremigen Konsistenz gehalten werden, so daß die Kollagenbiosynthese gesteigert und verbessert werden kann.

Erfindungsgemäß wird eine cremige, knebare Zubereitung aus den einzelnen Bestandteilen hergestellt. Dabei wird das Calciumhydroxid der Zubereitung gewohnlich in Mengen von 1-90 Gew.%, zweckmäßigerweise 10-70 Gew.%, vorzugsweise 20-60 Gew.%, jeweils bezogen auf das Gesamtgewicht der Zubereitung, zugegeben.

Das fette Öl vetarischen oder animalischen Ursprungs wird der Zusammensetzung zur Herbeiführung einer cremigen, knetbaren Konsistenz gewöhnlich in Mengen von 9-90 Gew.%, zweckmäßigerweise 10-60 Gew.%, vorzugsweise 20-40 Gew.%, jeweils bezogen auf das Gesamtgewicht der Zubereitung, zugegeben.

Der zwei- oder mehrwertige Alkohol wird der Zusammensetzung zur Herbeiführung einer cremigen, knetbaren Konsistenz gewöhnlich in Mengen von 1-40 Gew.%, zweckmäßigerweise 10-40 Gew.%, vorzugsweise 20-30 Gew.%, jeweils bezogen auf das Gesamtgewicht der Zubereitung, zugegeben.

Gegenstand einer bevorzugten Ausführungsform der vorliegenden Erfindung ist ein oben genanntes Gemisch, das zusätzlich MgO enthält. Das MgO kann dabei in Mengen von 1-90 Gew.%, zweckmäßigerweise 10-70 Gew.%, vorzugsweise 20-60 Gew.%, jeweils bezogen auf das Gesamtgewicht der Zubereitung, zugesetzt werden. Gegenwärtig wird jedoch davon ausgegangen, daß MgO vorzugsweise in kleineren Mengen von 10-20 Gew.% zugesetzt wird. MgO dient dabei im Knochenmaterial als Antacidum, das dem sauren Milieu im Knochen entgegenwirkt.

In dem erfindungsgemäßen Gemisch kann das Verhältnis Calciumhydroxid zu vegetarischem oder animalischem fettem Öl 5/1 bis 1/5, vorzugsweise 5/1 oder 1/1, betragen. Eine Abweichung vom bevorzugten Mischungsverhältnis kann jedoch durch die speziellen Gegebenheiten des Wundtraumas erforderlich sein.

Wenn das erfindungsgemäße Gemisch eine besonders geschmeidige und glatte Konsistenz aufweisen soll, kann ihm auch noch weiße Vaseline einverleibt werden. Gewöhnlich kann weiße Vasiline in Mengen von 1-60 Gew.%, zweckmäßigerweise in Mengen von 10-60 Gew.%, vorzugsweise in Mengen von 20-40 Gew.%, jeweils bezogen auf das Gesamtgewicht der Zubereitung, zugesetzt werden.

Obwohl üblicherweise der Knochenheilungs- oder -regenerationsprozeß nicht röntgenologisch überwacht zu werden braucht, kann dies doch in manchen Fällen angezeigt sein. Für diesen Fall kann dem erfindungsgemäßen Gemisch noch Bariumsulfat als Röntgenkontrastmittel einverleibt werden. Da jedoch das Bariumsulfat die Kollagenneubildung etwas schwächer ausfallen läßt, setzt man dem erfindungsgemäßen Gemisch im Bedarfsfall gerade so viel Bariumsulfat (beispielsweise 10-20 Gew.% bezogen auf das Gesamtgewicht der Zubereitung) zu, daß das Gemisch eben röntgensichtbar wird.

Die Applikation des erfindungsgemäßen Gemischs auf oder in das Knochentrauma kann - je nach seiner Konsistenz - mittels Spritzen, Spateln oder Pinseln erfolgen.

Für das erfindungsgemäße Gemisch gibt es zahlreiche Einsatzmöglichkeiten in der allgemeinen Chirurgie und Kieferchirurgie, Orthopädie, Implantologie, Traumatologie und dergleichen, da das erfindungsgemäße Gemisch auf oder in gewebige Knochentraumen, wie Frakturflächen, Bohrungen, Cavitäten und dergleichen applizierbar ist und am jeweiligen Applikationsort sofort eine in vivo-Kollagenneubildung induziert.

Da bekanntlich in manchen einschlägigen medizinischen Disziplinen mit metallischen Fixiermitteln gearbeitet wird, empfiehlt es sich in diesem Falle, die für das Einsetzen des Fixiermittels vorbereitete Bohrung vor dem Einsetzen des Fixiermittels mit dem erfindungsgemäßen Gemisch zu füllen und dann erst das Fixiermittel einzuführen. Dadurch kann man dem bei derartigen Maßnahmen unvermeidlichen primären Knochenschwund begegnen und dadurch die Ein- oder Anpassung des Fixiermittels in bzw. an das umgebende Knochengewebe und die Fixierung des Fixiermittels selbst durch das Knochengewebe beschleunigen.

Überschüssiges Gemisch stört hierbei im übrigen nicht, da es beim Einbringen des Fixiermittels in die mit dem Gemisch gefüllte Bohrung entweder wieder herausgedrückt wird oder in die Spongiosa diffundiert.

Es dürfte selbstverständlich sein, daß das erfindungsgemäße Gemisch bzw. seine Bestandteile sowohl steril verpackt als auch appliziert werden müssen.

In höchst überraschender Weise hat es sich auch noch gezeigt, daß das erfindungsgemäße Gemisch auch ohne Antibiotikum und/oder Corticoid-Unterstützung einer durch das Knochentrauma bedingten Entzündungsreaktion entgegenwirkt bzw. diese rasch zum Abklingen bringt. Seine einfache Zusammensetzung und ausgeprägte Wirksamkeit bei der in vivo-Kollagenneubildung unter gleichzeitiger Entzündungshemmung machen das erfindungsgemäße Gemisch zu einem in Zukunft unverzichtbaren Mittel in der Knochen-Traumatologie.

Die folgenden Beispiele sollen die Erfindung näher veranschaulichen.

### Beispiel 1

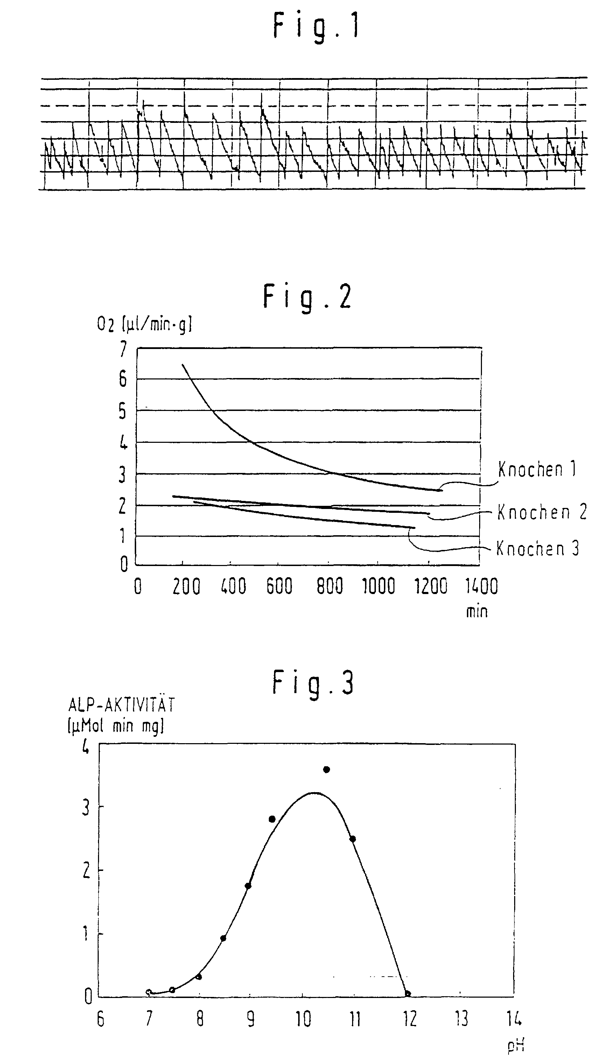

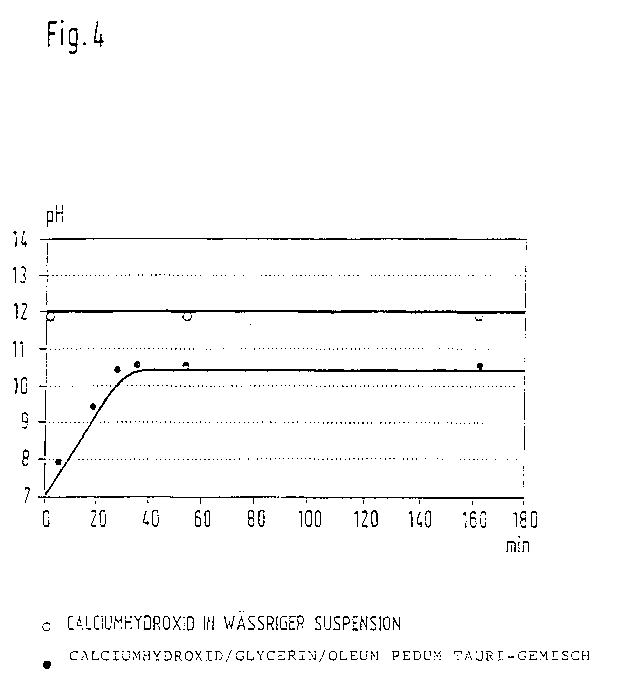

A) Zunächst soll die Wechselwirkung zwischen dem erfindungsgemäßen Gemisch und Gewebe aufgeklärt werden. So sind Daten über die Verteilung des erfindungsgemäßen Gemischs im Knochengewebe die Voraussetzung, um Hypothesen über einen möglichen Wirkmechanismus des Medikaments aufstellen zu können. Daher sind Experimente an Gewebekulturen sinnvoller als solche an Zellkulturen, da es erst in einer Gewebekultur möglich ist, Zell-Zell-Interaktionen zu studieren.
   1. Material und Methoden
      1.1 Gewebematerial
         Menschliches Knochengewebe, welches bei Osteotomien anfiel, wurde von Kliniken zur Verfügung gestellt.
         Embryonales Knochengewebe wurde aus 10 bis 17 Tage alten Hühnerembryonen (Gallus domesticus) gewonnen.
      1.2 Gewebekultur
         Unmittelbar nach Entnahme wurde das Gewebe in das Transportmedium überführe. Es wurden unter sterilen Bedingungen etwa 2 mm³ große Knochenfragmente präpariert und nach Bestimmung des Gewichts direkt für die Experimente eingesetzt.
         Für die Gewebekultur wurde die Earl'sche Modifikation des Minimal Essential Medium (MEM) nach Eagle mit 20 mM Hepes-Puffer verwendet.
         Dem Medium wird vor Versuchsbeginn 4% fötales Kälberserum und 1% Antibiotikumlösung (Penicillin/Streptomycin/Amphotericin B) zugesetzt, für die Markierungsexperimente zusätzlich 1 mM betaaminopropionidyl, 2 mM Na-Ascorbat und 2 bis 10 µg-Isotope (µC-Prolin). Kultiviert wird in 25 ml Erlenmeyer-Kolben bei 37°C im Schüttelwasserbad bei kleinster Frequenz.
      1.3 Bestimmung der Atmungsaktivität
         Die Atmungsaktivität ist ein sensibler Marker für die Stoffwechselaktivität des Gewebes. Schon geringste Änderungen des physiologischen Zustandes des Gewebes schlagen sich in einer meßbaren Änderung der Atmungsaktivität nieder.
         Für die Bestimmung der Atmungsaktivität wurde ein Clark-Sensor (Platin/Silber-Elektroden in gesättigter Kaliumchloridlösung) benutzt. Beim Anlegen einer Spannung von 0,8 V an die Elektrode ist der Sauerstoffreduktionsstrom direkt proportional zum Sauerstoffpartialdruck in der Meßlösung (Kulturmedium). Die Zufuhr von O₂-gesättigtem Medium bei Unterschreitung eines bestimmten Sauerstoffpartialdrucks und die Datenauswertung erfolgen rechnergesteuert.
         Knochengewebe besitzt typischerweise eine Atmungsaktivität von 2 - 3 µl O₂ x min⁻¹ x g⁻¹. Die Atmungsaktivität liegt damit größenordnungsmäßig im Bereich der Atmungsaktivität von ruhendem Muskelgewebe. Eine typische Atmungskurve für Knochengewebe zeigt Fig. 1. Der sägezahnartige Verlauf der Atmungskurve gemäß Fig. 1 ergibt sich aus der Tatsache, daß bei Unterschreitung eines bestimmten O₂-Partialdrucks in der Meßlösung frisches, O₂-gesättigtes Medium zugeführt wird.
         Fig. 2 zeigt durchschnittliche O₂-Verbrauchswerte aus drei Messungen. Der Sauerstoffverbrauch von embryonalem Knochengewebe (Gallus domesticus) wurde in einer Gewebekultur mit Hilfe des Clark-Sensors bestimmt. Der Sauerstoffverbrauch liegt zwischen 3 und 5 µl O² x min⁻¹ x g⁻¹. Die Atmungsaktivität nimmt im Laufe der Zeit um etwa 50 % ab, was bei Gewebekulturen durchaus normal ist.
      1.4 Enzymtests
         Ein Enzym, welches eng mit der Mineralisierung des Knochengewebes in Zusammenhang gebracht wird, ist alkalische Phosphatase. Dieses Enzym ist schon seit längerem charakterisiert, über die Funktion des Enzyms bei der Mineralisierung wird noch diskutiert. Da ein enger Zusammenhang zwischen der Osteoblastentätigkeit und der Aktivität von alkalischer Phosphatase besteht, kann alkalische Phosphatase als Marker der Osteoblastenaktivität angesehen werden. Bei Skelettwachstum im Jugendalter, bei der Knochenregeneration und bei Erkrankungen des Knochenstoffwechsels werden erhöhte Aktivitätswerte von alkalischer Phosphatase im Blutserum gefunden.
         Die Aktivität von alkalischer Phosphatase wurde in einem Rohextrakt bestimmt. Dafür wurden 500 mg Gewebe mit 1 ml Aufschlußpuffer versetzt und mit einem Messer zerkleinert. Anschließend wurden 500 mg Mahlperlen zugegeben und 20 min aufgeschlossen. Nach Zentrifugation wurde der Rohextrakt für die Messungen eingesetzt.
         Alkalische Phosphatase wird anhand des Umsatzes von p-Nitrophenylphosphat zu Nitrophenol und Phosphat nachgewiesen. Das bei der Hydrolyse entstehende Nitrophenol ist gelb und kann daher im Photometer bei einer Wellenlänge von 410 nm nachgewiesen werden.
         Die Fig. 3 zeigt die pH-Abhängigkeit der Aktivität von alkalischer Phosphatase. Die Aktivität von alkalischer Phosphatase aus Knochen wurde anhand des Umsatzes von p-Nitrophenylphosphat bestimmt. Das Aktivitätsmaximum liegt bei pH 10,5. Bei physiologischem pH von 7 besitzt alkalische Phosphatase nur etwa 1% der maximalen Aktivität.
      1.5 pH-Bestimmung
         Ein erfindungsgemäßes Gemisch, das Calciumhydroxid, Glycerin und oleum pedum tauri in Gewichtsanteilen von 30 Gew.%, 30 Gew.% bzw. 40 Gew.%, bezogen auf das Gesamtgewicht der Zubereitung, enthält, bzw. eine wäßrige Calciumhydroxid-Suspension wurde mit 30 ml Imidazol/HCl-Puffer (1 mM, pH 7) überschichtet, worauf der pH-Wert in der Lösung mit Hilfe einer pH-Elektrode kontinuierlich verfolgt wurde.
         Bei diesen Messungen zeigte es sich, daß ein Gemisch aus Calciumhydroxid und Glycerin in Oleum pedum tauri grundlegend andere Eigenschaften besitzt als Calciumhydroxid in wäßriger Suspension. Die Fig. 4 zeigt, daß eine wäßrige Calciumhydroxidsuspension einen sofortigen pH-Sprung auf pH 12 verursacht, und daß bei dem erfindungsgemäßen Glycerin/Calciumhydroxid/Oleum pedum tauri-Gemisch ein langsamer Anstieg bis zu einem pH-Wert von größer 10 erfolgt.
      1.6 Kollagenbestimmung
         Der Hauptteil der organischen Substanz im Knochen besteht - wie bereits erwähnt - aus Kollagen, einem Bindegewebsprotein. Wachstums- und Regenerationsprozeß des Knochens sind verbunden mit einer Kollagenneusynthese. Anschließend an die Synthese finden weitere intra- und extrazelluläre Kollagenprozesse statt. Unter Verwendung radioaktiver Kollagen-Vorstufen (¹⁴C-Prolin) ist es möglich, die Kollagensyntheserate in einer Gewebekultur genau zu quantifizieren. Somit ist es möglich, den Einfluß von Medikamenten auf die Kollagensynthese qualitativ und quantitativ zu erfassen.
         Der Kollagengesamtgehalt wird durch den sogenannten Hydroxyprolin-Test bestimmt. Die Aminosäure Hydroxyprolin kommt hauptsächlich im Kollagen vor, der Hydroxyprolingehalt in Fremdproteinen ist zu vernachlässigen. Nach Freisetzung der Aminosäuren aus den Proteinen durch Hydrolyse (16 h bei 116°C, 22% HCl) wird nach chemischer Modifizierung (Oxidation des 4-Hydroxyprolins zu Pyrrol) der Hydroxyprolingesamtgehalt im Testansatz durch eine spezifische Farbreaktion mit p-Dimethylaminobenzaldehyd quantifiziert.
      1.7 Bestimmung der Kollagensyntheserate
         Kollagen in Gewebe und Medium wird nach der durch Proff (1991) modifizierten Methode von Miller und Roths (vgl. E.J. Miller und R.H. Roths "Methods in Symol." 82:33 (1982)) gewonnen. Über mehrere Fällungsschritte mit anschließender Zentrifugation wird Kollagen über SDS-Gelelektrophorese und anschließendes Szintillationsmessen (Bestimmung der spezifischen Radioaktivität) quantifiziert. Zur Berechnung der Neusyntheserate wird der durch den Einbau von ¹⁴C-Prolin bestimmte Kollagenanteil, bezogen auf den Kollagengesamtgehalt, der durch den Hydroxyprolin-Test bestimmt wird, ermittelt.
         Durch eine Quantifizierung der Kollagenbiosynthese ist es möglich, den Einfluß von Calciumhydroxid-Präparaten auf die Knochenbildung zu untersuchen.
         Die Quantifizierung der Kollagenbiosynthese erfolgt - wie bei 1.7 angedeutet - nach der Technik der radioaktiven Markierung des Kollagens. Ein Bestandteil der Kollagenfaser ist die Aminosäure Prolin. Dem Kulturmedium wird eine genau definierte Menge von ¹⁴C-markiertem Prolin zugesetzt. Dieses Prolin wird in die während der Inkubation des Gewebes neu synthetisierten Proteine eingebaut. Nach der Trennung des Kollagens von anderen Proteinen ist es durch die Bestimmung der spezifischen Radioaktivität möglich, eine exakte quantitative Aussage über die Neusyntheserate des Kollagens zu machen.
         Die Isolierung des Kollagens geschieht über mehrere Fällungsschritte mit anschließender Zentrifugation und SDS-Gelelektrophorese. Bei der spezifischen Fällung des Kollagens werden die Kollagenfasern von anderen Proteinen getrennt, indem durch Zugabe von Natriumchlorid eine geeignete Salzkonzentration, bei der Nicht-Kollagen-Proteine zu einem großen Teil in Lösung bleiben, das Kollagen aber aus der Lösung als Niederschlag ausfällt, eingestellt wird. Durch anschließende Zentrifugation wird das Kollagen sedimentiert. Bei der SDS-Gelelektrophorese werden Proteine größenabhängig voneinander getrennt. Die Proteine wandern in einem elektrischen Feld durch eine Matrix aus einem hochvernetzten Polymer (Acrylamid). Kleine Proteine wandern schnell durch diese Matrix, da diese den kleinen Molekülen einen geringeren Widerstand entgegensetzt, große Proteine wandern langsamer, da ihre Beweglichkeit durch die Matrix stark behindert wird. Nach Färbung werden Proteine in diesem Gel als sogenannte "Banden" sichtbar. Durch interne Größenstandards können auf diese Weise Proteine anhand ihrer Größe identifiziert werden.
         Indem man interessierende Banden aus dem Gel herausschneidet, werden die Proteine einer weiteren Analyse, zum Beispiel einer Radioaktivitätsmessung, zugänglich.

### Extraktion von Kollagen:

Durch Zugabe von 3% Essigsäure wurde die Gewebekultur (vgl. 1.2) gestoppt. In Lösung gegangenes Kollagen wurde durch 2 M Natriumchlorid über Nacht bei 4°C gefällt und anschließend durch Zentrifugation (1 h, 24.000 x g, 4°C) gewonnen. Das Sediment wurde in 10 ml 3 %iger Essigsäure aufgenommen. Durch mechanische Desintegration der Gewebeblöcke wird neusynthetisiertes Kollagen, das sich innerhalb des Gewebeblockes befindet, in die Analyse miteinbezogen. Gewebereste wurden durch Zencrifugation (1 h, 45.000 x g, 4°C) gewonnen. Das Sediment wurde nach Solubilisierung gelelektrophoretisch aufgetrennt. Zur Kontrolle der Auftrennung der Proteine wurden sie im Gel angefärbt.

Das Gel wurde nach dem Lauf quer zur Laufrichtung in 5 mm breite Streifen zerschnitten, die Gelfragmente in Szintillationsgefäße überführt und im Szintillationszähler ausgezählt.

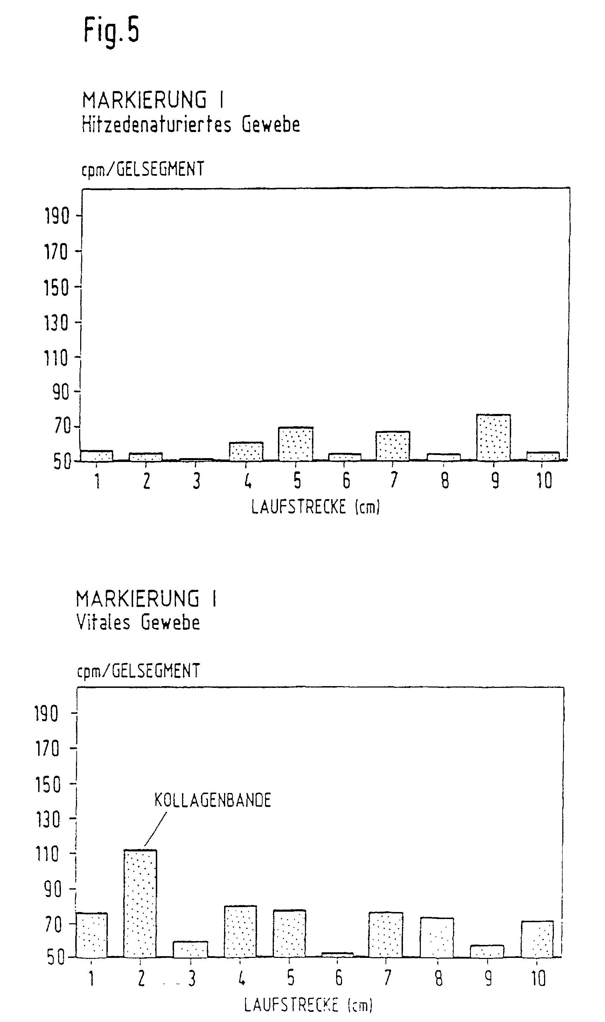

In Fig. 5 ist der Vergleich zwischen einem vitalen und einem hitzedenaturierten Gewebe gezeigt.

Dieser Vergleich ist in Fig. 5 anhand der Radioaktivitätsverteilung im Gel dargestellt. Kollagen als relativ großes Protein ist in dem Bereich 2 cm vom Start entfernt zu finden.

Der Kollagenbande, welche durch die Coomassie-Färbung nachweisbar wird, kann eine spezifische radioaktive Bande zugeordnet werden.

Fig. 5 Markierung I, Hüftkopf, Spongiosa, männlich, 45 Jahre: Unterschied der Kollagensyntheseleistung zwischen vitalem und hitzedenacuriertem Gewebe (CPM: Counts Per Minute). Gezeigt ist hier die Verteilung der Radioaktivität im Gel. Die Kollagenbande findet sich in einem Bereich, der etwa 2 cm vom Start entfernt liegt. Es tritt nur bei vitalem Gewebe eine Kollagenbande auf, das heißt der im Gel nachzuweisenden Radioaktivität entspricht eine Neusynthese des Kollagens während der Inkubation.

Das vitale Gewebe zeigt eine nachweisbare Kollagensyntheseleistung, das abgetötete Gewebe zeigt dagegen keine Stoffwechselaktivität mehr. Dies zeigt, daß das nachgewiesene radioaktive Kollagen tatsächlich auf eine Kollagenneusynthese in der Gewebekultur und nicht auf eine unspezifische Bindung radioaktiven Prolins an Proteine des Knochengewebes zurückzuführen ist. Für alle Versuche wurde eine vergleichbare Menge Gewebematerial eingesetzt (ca. 100 mg).

Es sind weitere radioaktiv markierte Banden geringerer Größe nachweisbar, wobei es sich um Abbauprodukte des Kollagens handeln kann. Der Abbau von Kollagen in der Gewebekultur ist vor allem bei Markierungsexperimenten über 4 Tage Inkubationsdauer zu beobachten. Zu einem geringen Teil ist auch radioaktiv markiertes Prolin im Gel vorhanden, welches durch die Fällungen nicht vollständig entfernt werden konnte.

Die Toleranz des Knochengewebes gegenüber schwach alkalischen pH-Werten ergibt sich aus einem Parallelexperiment zu dem in Fig. 5 erläuterten Experiment. Bei dem Parallelexperiment wurde der physiologische Hepes-Puffer des Kulturmediums (pH 7,4) durch einen Bicarbonatpuffer (pH 8,0) ersetzt. Hierbei zeigte es sich, daß die Alkalisierung des Kulturmediums auf pH 8,0 keinen meßbaren Unterschied der Kollagensyntheseleistung gegenüber pH 7,4 zur Folge hatte. Ab pH 8,5 ist keine gegenüber einer spontanen Kollagenneubildung erhöhte Kollagenneubildung mehr zu beobachten.

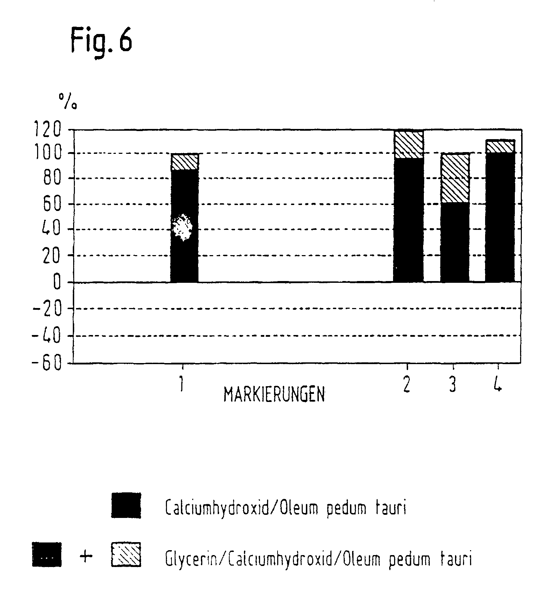

Die Figuren 6 bis 9 zeigen die Menge neusynthetisierten Kollagens in den Versuchsansätzen mit verschiedenen erfindungsgemäßen Gemischen im Vergleich zu Kontrollansätzen ohne dieses Gemisch bzw. ohne Mitverwendung eines zwei- oder mehrwertigen Alkohols. Die Differenz der neusynthetisierten Kollagenmenge in der Kontrolle und im Versuchsansatz ist in Prozent ausgedrückt. 0 % bedeutet keine gegenüber der Kontrolle vermehrte neusynthetisierte Kollagenmenge; 100 % bedeutet eine gegenüber der Kontrolle um das zweifache erhöhte Kollagenneubildung unter Einfluß eines erfindungsgemäßen Gemischs.

Aus Fig. 6 geht hervor, daß bei vier Experimenten unter dem Einfluß eines Glycerin/Calciumhydroxid/Oleum pedum tauri-Gemischs (Zusammensetzung: 30 Gew.% Ca(OH)₂, 30 Gew.% Glycerin, 40 Gew.% Oleum pedum tauri) die Kollagensynthese gegenüber der Kontrolle zwischen 100 und 120 % erhöht ist. Diese Steigerung ist signifikant, da experimentell bedingte Schwankungen der Kollagensyntheseleistung im Bereich von 10 bis 20 % liegen, die unter dem Einfluß des Glycerin/Calciumhydroxid/Oleum pedum tauri-Gemischs bestimmten Steigerungen dagegen zwischen 100 und 120 % liegen. Darüberhinaus ist auch eine Steigerung der Kollagensyntheseleistung gegenüber einem kein Glycerin enthaltenden Gemisch erkennbar.

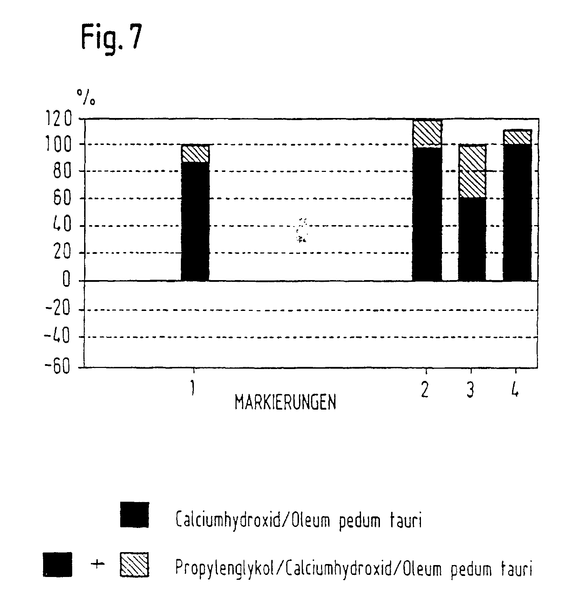

Aus Fig. 7 geht hervor, daß bei vier Experimenten unter dem Einfluß eines Propylenglykol/Calciumhydroxid/Oleum pedum tauri-Gemischs (Zusammensetzung: 30 Gew.% Ca(OH)₂, 30 Gew.% Propylenglykol, 40 Gew.% Oleum pedum tauri) die Kollagensynthese gegenüber der Kontrolle zwischen 100 und 120 % erhöht ist. Diese Steigerung ist signifikant, da experimentell bedingte Schwankungen der Kollagensyntheseleistung im Bereich von 10 bis 20 % liegen, die unter dem Einfluß des Propylenglykol/Calciumhydroxid/Oleum pedum tauri-Gemischs bestimmten Steigerungen dagegen zwischen 100 und 120 % liegen. Darüberhinaus ist auch eine Steigerung der Kollagensyntheseleistung gegenüber einem kein Propylenglykol enthaltenden Gemisch erkennbar.

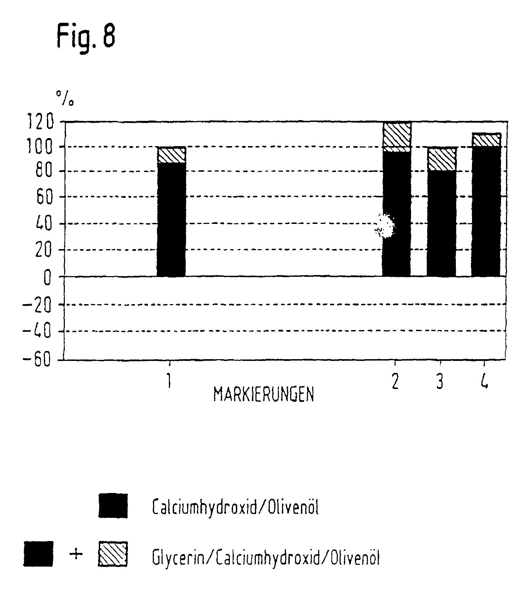

Aus Fig. 8 geht hervor, daß bei vier Experimenten unter dem Einfluß eines Glycerin/Calciumhydroxid/Olivenöl-Gemischs (Zusammensetzung: 30 Gew.% Ca(OH)₂, 30 Gew.% Glycerin, 40 Gew.% Olivenöl) die Kollagensynthese gegenüber der Kontrolle zwischen 100 und 120 erhöht ist. Diese Steigerung ist signifikant, da experimentell bedingte Schwankungen der Kollagensyntheseleistung im Bereich von 10 bis 20 % liegen, die unter dem Einfluß des Glycerin/Calciumhydroxid/Olivenöl-Gemischs bestimmten Steigerungen dagegen zwischen 100 und 120 % liegen.

Darüberhinaus ist auch eine Steigerung der Kollagensyntheseleistung gegenüber einem kein Glycerin enthaltenden Gemisch erkennbar.

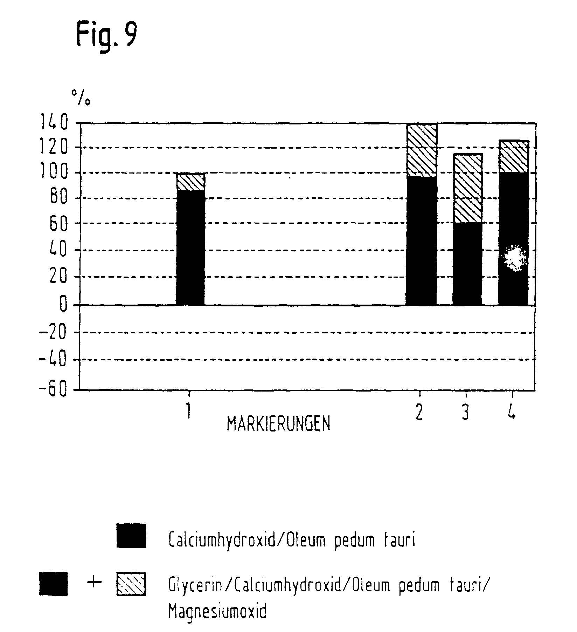

Aus Fig. 9 geht hervor, daß bei vier Experimenten unter dem Einfluß eines Glycerin/Calciumhydroxid/Magnesiumoxid/Oleum pedum tauri-Gemischs (Zusammensetzung: 20 Gew.% Ca(OH)₂, 20 Gew.% Glycerin, 20 Gew.% Magnesiumoxid, 40 Gew.% Oleum pedum tauri) die Kollagensynthese gegenüber der Kontrolle zwischen 100 und 140 % erhöht ist. Diese Steigerung ist signifikant, da experimentell bedingte Schwankungen der Kollagensyntheseleistung im Bereich von 10 bis 20 % liegen, die unter dem Einfluß des Glycerin/Calciumhydroxid/Magnesiumoxid/Oleum pedum tauri-Gemischs bestimmten Steigerungen dagegen zwischen 100 und 140 % liegen. Darüberhinaus ist auch eine Steigerung der Kollagensyntheseleistung gegenüber einem kein Glycerin und MgO enthaltenden Gemisch erkennbar.

### Beispiel 2

In einem Haltbarkeitstest, bei dem die im folgenden angegebenen Gemische nach Formulierung zu einer knetbaren bzw. cremigen Masse bei Raumtemperatur und Umgebungsluftfeuchtigkeit gelagert wurden, wurde festgestellt, daß die Beibehaltung der gewünschten Konsistenz des Gemischs bei Mitverwendung eines zwei- oder mehrwertigen Alkohols im Vergleich zu Proben ohne den Alkohol um mindestens 50% der Haltbarkeitszeitspanne verlängert werden kann.

Verwendete Gemische:
1) Glycerin/Calciumhydroxid/Oleum pedum-Gemisch (30 Gew.%/30 Gew.%/40 Gew.%)
2) Propylenglykol/Calciumhydroxid/Oleum pedum tauri-Gemisch (30 Gew.%/30 Gew.%/40 Gew.%) und
3) Glycerin/Calciumhydroxid/Olivenöl-Gemisch (30 Gew.%/30 Gew.%/40 Gew.%)

| 1. Gemisch | ohne Glycerin | mit Glycerin |
|---|---|---|
| knetbare Konsistenz | 6 Monate | 12 Monate |
| cremige Konsistenz | 12 Monate | 18 Monate |
| | | |

| 2. Gemisch | ohne Propylenglykol | mit Propylenglykol |
|---|---|---|
| knetbare Konsistenz | 6 Monate | 12 Monate |
| cremige Konsistenz | 12 Monate | 18 Monate |
| | | |

| 3. Gemisch | ohne Olivenöl | mit Olivenöl |
|---|---|---|
| knetbare Konsistenz | 6 Monate | 12 Monate |
| cremige Konsistenz | 12 Monate | 18 Monate |

## Patentansprüche

1. Pharmazeutische Zubereitung, die Calciumhydroxid, ein fettes Öl vegetarischen oder animalischen Ursprungs sowie gegebenenfalls pharmazeutisch verträgliche Hilfsstoffe enthält, **dadurch gekennzeichnet, daß** sie ferner einen zwei- oder mehrwertigen Alkohol enthält.

2. Pharmazeutische Zubereitung nach Anspruch 1, **dadurch gekennzeichnet, daß** das Volumenverhältnis Calciumhydroxid, vegetarisches oder animalisches fettes Öl 5/1 bis 1/5 beträgt.

3. Pharmazeutische Zubereitung nach Anspruch 1, **dadurch gekennzeichnet, daß** das Volumenverhältnis Calciumhydroxid, vegetarisches oder animalisches fettes Öl 1/1 beträgt.

4. Pharmazeutische Zubereitung nach Anspruch 1, **dadurch gekennzeichnet, daß** das Volumenverhältnis Calciumhydroxid, vegetarisches oder animalisches fettes Öl 5/1 beträgt.

5. Pharmazeutische Zubereitung nach einem der Ansprüche 1 bis 4, **dadurch gekennzeichnet, daß** sie zusätzlich Bariumsulfat enthält.

6. Pharmazeutische Zubereitung nach einem der Ansprüche 1 bis 5, **dadurch gekennzeichnet, daß** sie zusätzlich weiße Vaseline enthält.

7. Pharmazeutische Zubereitung nach einem der vorhergehenden Ansprüche, **dadurch gekennzeichnet, daß** sie zusätzlich MgO enthält.

8. Pharmazeutische Zubereitung nach einem der Ansprüche 1 bis 7, **dadurch gekennzeichnet, daß** es sich bei dem zwei- oder mehrwertigen Alkohol um Glycerin handelt.

9. Pharmazeutische Zubereitung nach einem der Ansprüche 1 bis 8, **dadurch gekennzeichnet, daß** es sich bei dem vegetarischen oder animalischen fetten Öl um Oleum pedum tauri handelt.

10. Pharmazeutische Zubereitung nach einem der Ansprüche 1 bis 8, **dadurch gekennzeichnet, daß** es sich bei dem vegetarischen oder animalischen fetten Öl um Olivenöl handelt.

11. Verfahren zur Herstellung einer pharmazeutischen Zubereitung nach einem der vorhergehenden Ansprüche durch Einmischen des Calciumhydroxids und eines zwei- oder mehrwertigen Alkohols in einem fetten Öl vegetarischen oder animalischen Ursprungs.

12. Verwendung einer pharmazeutischen Zubereitung, die Calciumhydroxid, einen zwei- oder mehrwertigen Alkohol und ein fettes Öl vegetarischen oder animalischen Ursprungs sowie gegebenenfalls pharmazeutisch verträgliche Hilfsstoffe enthält, bei der Herstellung eines Medikaments zur Förderung der Kollagenneubildung in vivo.

## Claims

1. A pharmaceutical preparation which comprises calcium hydroxide, a fixed oil of vegetable or animal origin and, where appropriate, pharmaceutically acceptable excipients, which additionally comprises a dihydric or polyhydric alcohol.

2. A pharmaceutical preparation as claimed in claim 1, wherein the ratio by volume of calcium hydroxide and vegetable or animal fixed oil is 5/1 to 1/5.

3. A pharmaceutical preparation as claimed in claim 1, wherein the ratio by volume of calcium hydroxide and vegetable or animal fixed oil is 1/1.

4. A pharmaceutical preparation as claimed in claim 1, wherein the ratio by volume of calcium hydroxide and vegetable or animal fixed oil is 5/1.

5. A pharmaceutical preparation as claimed in any of claims 1 to 4, which additionally comprises barium sulfate.

6. A pharmaceutical preparation as claimed in any of claims 1 to 5, which additionally comprises white petrolatum.

7. A pharmaceutical preparation as claimed in any of the preceding claims, which additionally comprises MgO.

8. A pharmaceutical preparation as claimed in any of claims 1 to 7, wherein the dihydric or polyhydric alcohol is glycerol.

9. A pharmaceutical preparation as claimed in any of claims 1 to 8, wherein the vegetable or animal fixed oil is neatsfoot oil.

10. A pharmaceutical preparation as claimed in any of claims 1 to 8, wherein the vegetable or animal fixed oil is olive oil.

11. A process for the production of a pharmaceutical preparation as claimed in any of the preceding claims by mixing calcium hydroxide and a dihydric or polyhydric alcohol into a fixed oil of vegetable or animal origin.

12. The use of a pharmaceutical preparation which comprises calcium hydroxide, a dihydric or polyhydric alcohol and a fixed oil of vegetable or animal origin and, where appropriate, pharmaceutically acceptable excipients for producing a medicine for promoting collagen regeneration in vivo.

## Revendications

1. Préparation pharmaceutique, qui contient de l'hydroxyde de calcium, une huile grasse d'origine végétale ou animale ainsi que, le cas échéant, un adjuvant pharmaceutiquement acceptable, **caractérisée en ce qu'**elle contient en outre un alcool bivalent ou plurivalent.

2. Préparation pharmaceutique selon la revendication 1, **caractérisée en ce que** le rapport volumique de l'hydroxyde de calcium et de l'huile grasse animale ou végétale se situe de 5/1 à 1/5.

3. Préparation pharmaceutique selon la revendication 1, **caractérisée en ce que** le rapport volumique de l'hydroxyde de calcium et de l'huile grasse animale ou végétale est de 1/1.

4. Préparation pharmaceutique selon la revendication 1, **caractérisée en ce que** le rapport volumique de l'hydroxyde de calcium et de l'huile grasse animale ou végétale est de 5/1.

5. Préparation pharmaceutique selon l'une quelconque des revendications 1 à 4, **caractérisée en ce qu'**elle contient en outre du sulfate de baryum.

6. Préparation pharmaceutique selon l'une quelconque des revendications 1 à 5, **caractérisée en ce qu'**elle contient en outre de la vaseline blanche.

7. Préparation pharmaceutique selon l'une quelconque des revendications précédentes, **caractérisée en ce qu'**elle contient en outre du MgO.

8. Préparation pharmaceutique selon l'une quelconque des revendications 1 à 7, **caractérisée en ce qu'**il s'agit, en ce qui concerne l'alcool bivalent ou plurivalent, de glycérine.

9. Préparation pharmaceutique selon l'une quelconque des revendications 1 à 8, **caractérisée en ce qu'**il s'agit, en ce qui concerne l'huile grasse animale ou végétale, d'oleum pedum tauri.

10. Préparation pharmaceutique selon l'une quelconque des revendications 1 à 8, **caractérisée en ce qu'**il s'agit, en ce qui concerne l'huile grasse animale ou végétale, d'huile d'olive.

11. Procédé de fabrication d'une préparation pharmaceutique selon l'une quelconque des revendications précédentes, par mélange d'hydroxyde de calcium et d'un alcool bivalent ou plurivalent dans une huile grasse d'origine végétale ou animale.

12. Utilisation d'une préparation pharmaceutique, qui contient de l'hydroxyde de calcium, un alcool bivalent ou plurivalent et une huile grasse d'origine végétale ou animale ainsi que, le cas échéant, un adjuvant pharmaceutiquement acceptable pour la fabrication d'un médicament destiné à promouvoir la néoformation de collagène in vivo.
